# EUROPEAN PATENT APPLICATION

(11) **EP 4 230 248 A1**
(43) Date of publication of application: **23.08.2023**
(21) Application number: 21882797.0
(22) Date of filing: 19.10.2021
(51) Int. Cl.: A61M 21/02, F24F 11/80, F24F 110/10, F24F 110/20, F24F 110/60, F24F 130/40

(54) **NAP ENVIRONMENT DECISION SYSTEM**

(30) Priority: 19.10.2020 JP 2020175270
(71) Applicant: Daikin Industries, Ltd., Osaka-shi, Osaka 530-8323 (JP); The University of Electro-Communications, Chofu-shi, Tokyo 182-8585 (JP)
(72) Inventor: OHGA, Takahiro, Osaka-shi, Osaka 530-0001 (JP); ASHIKAGA, Tomoyoshi, Osaka-shi, Osaka 530-0001 (JP); TSUJIMOTO, Yukako, Osaka-shi, Osaka 530-0001 (JP); OKEMA, Chiharu, Osaka-shi, Osaka 530-0001 (JP); IIDA, Hikaru, Osaka-shi, Osaka 530-0001 (JP); KAWABATA, Rieko, Osaka-shi, Osaka 530-0001 (JP); SAWADA, Ryuunosuke, Osaka-shi, Osaka 530-0001 (JP); TAKADAMA, Keiki, Chofu-shi, Tokyo 182-8585 (JP)
(74) Representative: Global IP Europe Patentanwaltskanzlei
(86) International application number: PCT/JP2021/038515
(87) International publication number: WO 2022/085656

(57) **Abstract**

An environment that is suitable for a subject during a nap is provided. A nap environment determination system (1) is a system for determining an environment condition during a nap, and includes a pre-nap information acquisition unit and a determination unit. The pre-nap information acquisition unit acquires pre-nap information regarding a subject who takes a nap before the subject takes the nap. The determination unit determines an environment condition for adjusting a sleep depth, based on the pre-nap information.

## Description

### Technical Field

The present disclosure relates to a nap environment determination system.

### Background Art

Conventionally, PTL 1 (Japanese Patent No. 4466654) discloses a sleep state improvement system and a sleep state improvement method capable of improving the sleep state of a user also while the user is away from home.

### Summary of Invention

### <Technical Problem>

However, in PTL 1 noted above, a case in which the user takes a nap is not considered in detail. Therefore, the sleep environment provided by the system and method of PTL 1 noted above is not necessarily an environment suitable for the user who takes a nap.

### <Solution to Problem>

A nap environment determination system of a first aspect is a system for determining an environment condition during a nap and includes a pre-nap information acquisition unit and a determination unit. The pre-nap information acquisition unit acquires pre-nap information regarding a subject who takes a nap before the subject takes the nap. The determination unit determines an environment condition for adjusting a sleep depth, based on the pre-nap information.

In the nap environment determination system, which is a system for determining an environment condition during a nap, the determination unit determines the environment condition for adjusting the sleep depth, based on the pre-nap information. The pre-nap information is information regarding the subject who takes a nap and is information acquired before the subject takes the nap. According to this configuration, the nap environment determination system can determine the environment condition during a nap, based on the environment condition for adjusting the sleep depth which is determined by the determination unit. Therefore, the nap environment determination system according to the first aspect can provide an environment that is suitable for the subject during a nap.

A nap environment determination system of a second aspect is the system of the first aspect and further includes a creation unit. The creation unit creates a nap plan related to the environment condition for adjusting the sleep depth, based on a determination of the determination unit. The nap plan includes the number of times the environment condition is adjusted, a time at which the environment condition is adjusted, a target transition of a sleep state switched by adjustment of the environment condition, and a control parameter for adjusting the environment condition.

In the nap environment determination system, the creation unit creates the nap plan related to the environment condition for adjusting the sleep depth, based on the determination of the determination unit. According to this configuration, the nap environment determination system can determine the environment condition during a nap, based on the nap plan created by the creation unit. Therefore, the nap environment determination system according to the second aspect can provide an environment that is suitable for the subject during a nap.

A nap environment determination system of a third aspect is the system of the first aspect or the second aspect and further includes an intra-nap information acquisition unit and an update unit. The intra-nap information acquisition unit acquires intra-nap information regarding the subject who is taking a nap while the subject is taking the nap. The update unit updates the nap plan, based on the intra-nap information while the subject is taking the nap.

In the nap environment determination system, the update unit updates the nap plan, based on the intra-nap information while the subject is taking a nap. The intra-nap information is information regarding the subject who is taking a nap and is information acquired while the subject is taking the nap. According to this configuration, the nap environment determination system can determine the environment condition during a nap, based on the nap plan that has been updated while the subject is taking the nap. Therefore, the nap environment determination system according to the third aspect can provide an environment that is more suitable for the subject during a nap.

A nap environment determination system of a fourth aspect is the system of any one of the first aspect to the third aspect, wherein the pre-nap information includes at least one of a sleep onset time of a previous day, an average sleep onset time, a wake-up time of the previous day, an average wake-up time, and drowsiness, of the subject. In addition, the pre-nap information includes at least one of a gender, an age, and a desired nap time, of the subject.

In the nap environment determination system, information specific to the subject is acquired as the pre-nap information. According to this configuration, the nap environment determination system can determine the environment condition during a nap, based on the characteristics of the subject. Therefore, the nap environment determination system according to the fourth aspect can provide an environment that is more suitable for the subject during a nap.

A nap environment determination system of a fifth aspect is the system of any one of the first aspect to the fourth aspect, wherein the pre-nap information further includes at least one of a level of fatigue, a sleeping time of a previous day, and an average sleeping time, of the subject.

In the nap environment determination system, information specific to the subject is acquired as the pre-nap information. According to this configuration, the nap environment determination system can determine the environment condition during a nap, based on the characteristics of the subject. Therefore, the nap environment determination system according to the fifth aspect can provide an environment that is more suitable for the subject during a nap.

A nap environment determination system of a sixth aspect is the system of any one of the first aspect to the fifth aspect, wherein the pre-nap information is acquired from at least one of a manual input by the subject and a pre-nap information acquisition device that is a device for acquiring information about the subject.

A nap environment determination system of a seventh aspect is the system of any one of the third aspect to the sixth aspect, wherein the intra-nap information includes at least one of a heartbeat, an electrocardiogram, a pulse wave, a brain wave, breathing, a blood pressure, a body temperature, and a body motion. In addition, the intra-nap information includes at least one of a temperature, a humidity, an airflow, an illuminance, a noise, an odor, a temperature of bedding, and a gradient of the bedding, in a space in which the subject who is taking a nap is present. The intra-nap information is acquired by an intra-nap information acquisition device installed in the space in which the subject who is taking the nap is present.

In the nap environment determination system, biological information specific to the subject and information specific to the space in which the subject is present are acquired as the intra-nap information. In addition, the intra-nap information is information acquired while the subject is taking a nap. According to this configuration, the nap environment determination system can determine the environment condition during a nap, based on the current characteristics of the subject and the current characteristics of the space in which the subject is present. Therefore, the nap environment determination system according to the seventh aspect can provide an environment that is more suitable for the subject during a nap.

A nap environment determination system of an eighth aspect is the system of any one of the second aspect to the seventh aspect, wherein the sleep state includes a wakeful state, a REM sleep state, a light sleep state, and a deep sleep state.

A nap environment determination system of a ninth aspect is the system of any one of the second aspect to the eighth aspect, wherein the environment condition includes at least one of a thermal environment, a lighting environment, an intra-bedding thermal environment, a bedding position, and a sound environment, in a space in which the subject who is taking a nap is present.

A nap environment determination system of a tenth aspect is the system of any of the second aspect to the ninth aspect and further includes an environment condition adjustment device and an actuator. The environment condition adjustment device is a device for adjusting the environment condition, based on the nap plan. The actuator controls the environment condition adjustment device. The environment condition adjustment device includes an air-conditioning apparatus and bedding having a temperature adjustment unit.

The nap environment determination system further includes an environment condition adjustment device that is a device for adjusting the environment condition, based on the nap plan, and an actuator that controls the environment condition adjustment device. According to this configuration, the environment condition can be adjusted by the environment condition adjustment device.

### Brief Description of Drawings

[Fig. 1] Fig. 1 is a conceptual diagram of a nap environment determination system.
[Fig. 2] Fig. 2 is a block diagram illustrating a configuration of a management apparatus.
[Fig. 3] Fig. 3 is a diagram illustrating a nap plan.
[Fig. 4] Fig. 4 is a diagram illustrating an example in which the sleep depth is adjusted by adjusting a nap environment condition.
[Fig. 5] Fig. 5 is a flowchart illustrating an operation flow of the nap environment determination system.
[Fig. 6] Fig. 6 is a diagram illustrating a nap report.

### Description of Embodiments

Hereinafter, embodiments of a nap environment determination system 1 according to the present disclosure will be described with reference to the drawings, as appropriate. However, unnecessarily detailed description may be omitted. For example, detailed description of an already well-known matter or redundant description of a substantially identical configuration may be omitted. This is to avoid making unnecessarily redundant descriptions below and to facilitate understanding by those skilled in the art.

Note that the drawings are not necessarily precise illustrations. In addition, in the drawings, substantially the same configurations are denoted by the same reference numerals, and overlapping descriptions are omitted or simplified.

### (1) Overall configuration of nap environment determination system 1

A nap environment determination system 1 according to the present embodiment is a system that determines an environment condition during a nap. In the present embodiment, the environment condition during a nap includes at least one of a temperature, a humidity, an airflow, an illuminance, a noise, a temperature of bedding, and a gradient of the bedding, in a space in which the subject who is taking a nap is present. The nap environment determination system 1 determines the environment condition during a nap so that the sleep depth during the nap increases, so that the sleep depth during the nap decreases, or so that the sleep depth during the nap is maintained. Details will be described below.

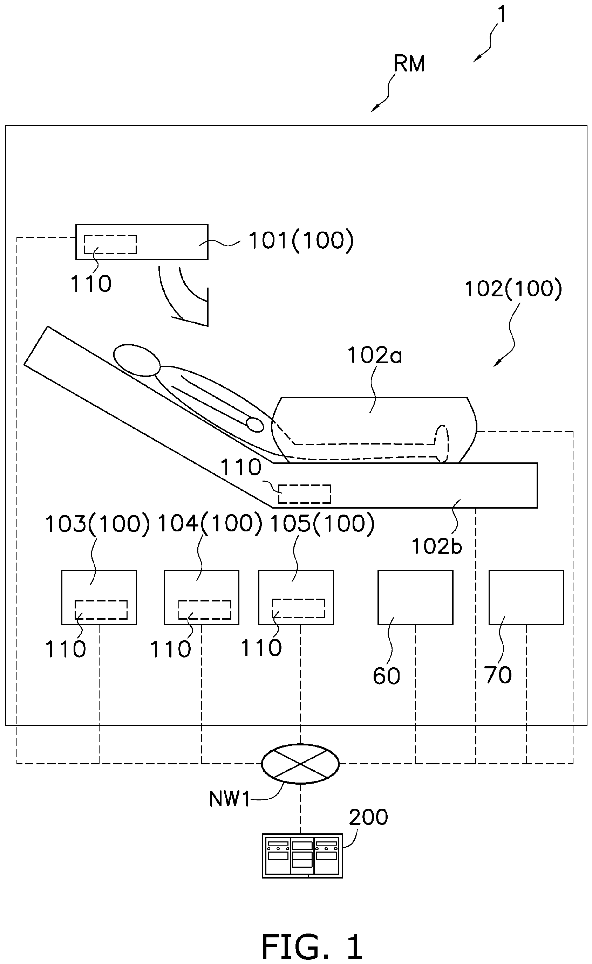

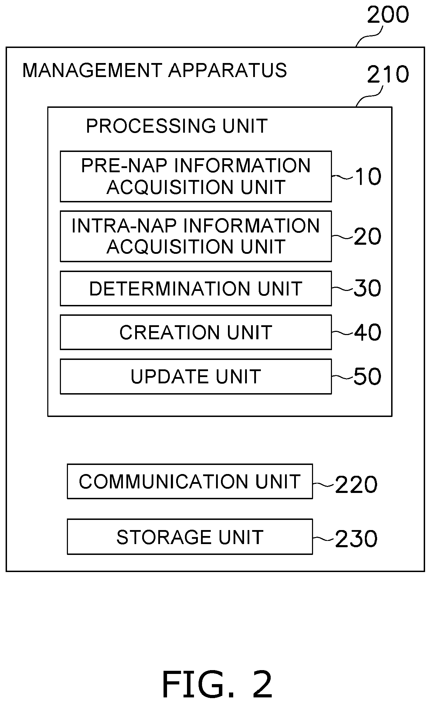

As illustrated in Figs. 1 and 2, the nap environment determination system 1 according to the present embodiment mainly includes a pre-nap information acquisition unit 10, an intra-nap information acquisition unit 20, a determination unit 30, a creation unit 40, an update unit 50, a pre-nap information acquisition device 60, an intra-nap information acquisition device 70, an environment condition adjustment device 100, and actuators 110.

Although details will be described below, in the nap environment determination system 1 according to the present embodiment, the pre-nap information acquisition unit 10, the intra-nap information acquisition unit 20, the determination unit 30, the creation unit 40, and the update unit 50 are included in a management apparatus 200 (see Fig. 2).

As illustrated in Fig. 1, the subject takes a nap on the bedding 102 in a space RM in which the pre-nap information acquisition device 60, the intra-nap information acquisition device 70, and the like are installed. Note that in the present embodiment, a "nap" refers to a sleep for 10 minutes or more and three hours or less, for example. In addition, it is assumed that a "nap" is started at a time point when the subject comes to be present on the bedding 102. Therefore, as long as the subject is present on the bedding 102, it is considered that the subject is taking a nap even if the subject is in a wakeful state.

In the nap environment determination system 1 according to the present embodiment, the pre-nap information regarding the subject who takes a nap is acquired by the pre-nap information acquisition device 60 before the subject takes the nap. The pre-nap information includes at least one of a sleep onset time of a previous day, an average sleep onset time, a wake-up time of the previous day, an average wake-up time, drowsiness, and a desired nap time, of the subject. In addition, the pre-nap information includes at least one of a gender, an age, and a desired nap time, of the subject. Further, the pre-nap information includes at least one of a level of fatigue, a sleeping time of the previous day, and an average sleeping time, of the subject. The determination unit 30 of the nap environment determination system 1 determines an environment condition for adjusting the sleep depth, based on the pre-nap information. The creation unit 40 of the nap environment determination system 1 creates a nap plan related to the environment condition for adjusting the sleep depth, based on the determination of the determination unit 30. The nap environment determination system 1 adjusts the environment condition, based on the nap plan created by the creation unit 40.

In addition, in the nap environment determination system 1 according to the present embodiment, intra-nap information regarding the subject who is taking a nap is acquired by the intra-nap information acquisition device 70 while the subject is taking the nap. The intra-nap information includes at least one of a heartbeat, an electrocardiogram, a pulse wave, a brain wave, breathing, a blood pressure, a body temperature, and a body motion, of the subject. In addition, the intra-nap information includes at least one of a temperature, a humidity, an airflow, an illuminance, a noise, an odor, a temperature of the bedding, and a gradient of the bedding, in a space RM in which the subject who is taking a nap is present. The update unit 50 of the nap environment determination system 1 updates the nap plan, based on these pieces of intra-nap information. The nap environment determination system 1 adjusts the environment condition based on the updated nap plan. By adjusting the environment condition, the sleep depth of the subject is adjusted.

### (2) Detailed configuration of nap environment determination system 1

### (2-1) Pre-nap information acquisition device 60

The pre-nap information acquisition device 60 is a device that acquires pre-nap information. The pre-nap information acquisition device 60 according to the present embodiment is, for example, a wearable device such as a smart watch or smart glasses. For example, the pre-nap information acquisition device 60 acquires the pre-nap information by means of a sensor, such as a vital sensor built in the pre-nap information acquisition device 60. Alternatively, the pre-nap information acquisition device 60 acquires the pre-nap information by, for example, receiving a manual input from the subject. Alternatively, the pre-nap information acquisition device 60 acquires the pre-nap information by communicating with an information terminal (for example, a computer installed at home or the workplace of the subject), which is not illustrated and in which the pre-nap information is recorded, via a communication network NW1 or the like.

Note that the pre-nap information acquisition device 60 according to the present embodiment is not limited to a single device and may be configured by combining a plurality of devices. In addition, the pre-nap information acquisition device 60 is not limited to a wearable device and may be changed, as appropriate. Therefore, the pre-nap information acquisition device 60 may be, for example, a smartphone or the like.

### (2-2) Intra-nap information acquisition device 70

The intra-nap information acquisition device 70 is a device that acquires intra-nap information. The intra-nap information acquisition device 70 according to the present embodiment is, for example, but not limited to, a sensor unit installed in the space RM in which the subject who is taking a nap is present. More specifically, the intra-nap information acquisition device 70 according to the present embodiment includes any one of a vital sensor, a motion sensor, an electroencephalograph, and a heart rate meter. In addition, the intra-nap information acquisition device 70 according to the present embodiment further includes any one of an air temperature sensor, an air humidity sensor, an airflow sensor, an illuminance sensor, a sound pressure sensor that detects a noise level, an odor sensor that detects an amount of an odor component contained in air, a temperature sensor that detects a temperature of the bedding 102, and a gradient sensor that detects a gradient of the bedding 102.

Note that the intra-nap information acquisition device 70 according to the present embodiment is not limited to a single device and may be configured by combining a plurality of devices. The intra-nap information acquisition device 70 is not limited to the sensor unit described above and may be selected, as appropriate.

### (2-3) Environment condition adjustment device 100

The environment condition adjustment device 100 is a device that adjusts the environment condition. As described above, the environment condition includes at least one of a thermal environment, a lighting environment, an intra-bedding thermal environment, a bedding position, an odor environment, and a sound environment, in the space RM in which the subject who is taking a nap is present.

The environment condition adjustment device 100 includes an air-conditioning apparatus 101 and the bedding 102, which includes a temperature adjustment unit. The environment condition adjustment device 100 may also include a lighting device 103, a speaker 104, and a scent dispenser 105. The air-conditioning apparatus 101 is a device capable of adjusting the temperature and humidity of air in the space RM and is a device capable of adjusting the thermal environment in the space RM in which the subject who is taking a nap is present. The bedding 102 is a device including, for example, an electric blanket 102a as the temperature adjustment unit and a bed 102b whose position can be changed and is a device capable of adjusting an intra-bedding thermal environment and a bedding position in the space RM in which the subject who is taking a nap is present. The lighting device 103 is, for example, a device capable of changing illuminance and is a device capable of adjusting a lighting environment in the space RM in which the subject who is taking a nap is present. The speaker 104 is, for example, a device capable of adjusting a sound volume and is a device capable of adjusting a sound environment in the space RM in which the subject who is taking a nap is present. The scent dispenser 105 is a device that contains, for example, a plurality of scents and can adjust the odor environment in the space RM in which the subject who is taking a nap is present by generating a scent using one scent and/or a plurality of scents.

### (2-4) Actuator 110

The nap environment determination system 1 includes actuators 110 that control the environment condition adjustment device 100. More specifically, the nap environment determination system 1 includes the actuators 110 that control the operation of the environment condition adjustment device 100. The actuators 110 according to the present embodiment are built in the air-conditioning apparatus 101, the bedding 102, and the like as the environment condition adjustment device 100. By controlling the actuators 110, the nap environment determination system 1 can control at least one of a thermal environment, a lighting environment, an intra-bedding thermal environment, a bedding position, an odor environment, and a sound environment, in the space RM in which the subject who is taking a nap is present.

### (2-5) Management apparatus 200

The management apparatus 200 according to the present embodiment is, for example, a computer apparatus including a processing unit 210, a communication unit 220, and a storage unit 230. In the present embodiment, the management apparatus 200 is installed in a space different from the space RM. The management apparatus 200 is a device apparatus that manages the environment condition of the space RM. The management apparatus 200 is connected by the function of the communication unit 220 to each of the pre-nap information acquisition device 60, the intra-nap information acquisition device 70, and the environment condition adjustment device 100 via the communication network NW1.

Hereinafter, the management apparatus 200 constituting the nap environment determination system 1 will be described with reference to the drawings. Note that the management apparatus 200 may not have some or all of the functions described in the present embodiment. For example, some or all of the functions of the management apparatus 200 described below may be realized by a server or the like installed in a place different from the management apparatus 200.

### (3) Detailed configuration of management apparatus 200

The configuration of the management apparatus 200 constituting the nap environment determination system 1 will be described in detail. Fig. 2 is a block diagram illustrating the configuration of the management apparatus 200. As illustrated in Fig. 2, the management apparatus 200 according to the present embodiment includes the processing unit 210, the communication unit 220, and the storage unit 230.

### (3-1) Processing unit 210

The processing unit 210 includes a CPU. The CPU of the processing unit 210 reads and executes various programs stored in the storage unit 230 to function as the pre-nap information acquisition unit 10, the intra-nap information acquisition unit 20, the determination unit 30, the creation unit 40, and the update unit 50.

### (3-1-1) Pre-nap information acquisition unit 10

The pre-nap information acquisition unit 10 acquires pre-nap information. Specifically, the pre-nap information acquisition unit 10 acquires the pre-nap information from the pre-nap information acquisition device 60 via the communication network NW1, by means of the communication unit 220 described below. Note that the pre-nap information acquisition unit 10 may acquire the pre-nap information by receiving a manual input from the subject. In other words, the pre-nap information acquisition unit 10 may acquire the pre-nap information without using the pre-nap information acquisition device 60.

### (3-1-2) Intra-nap information acquisition unit 20

The intra-nap information acquisition unit 20 acquires intra-nap information. Specifically, the intra-nap information acquisition unit 20 acquires the intra-nap information from the intra-nap information acquisition device 70 via the communication network NW1, by means of the communication unit 220 described below.

### (3-1-3) Determination unit 30

The determination unit 30 determines the environment condition for adjusting the sleep depth of the subject, based on the pre-nap information. In other words, the determination unit 30 determines how to adjust the environment condition to adjust the sleep depth of the subject. Note that the adjustment of the sleep depth means any one of increasing the sleep depth, decreasing the sleep depth, and maintaining the sleep depth. Therefore, the determination unit 30 can be rephrased as a functional unit that determines how to adjust the environment condition to increase, decrease, and maintain the sleep depth of the subject.

### (3-1-4) Creation unit 40

The creation unit 40 creates a nap plan related to the environment condition for adjusting the sleep depth, based on the determination of the determination unit 30. The nap plan includes the number of times the environment condition is adjusted, a time at which the environment condition is adjusted, a target transition of a sleep state switched by adjustment of the environment condition, and a control parameter for adjusting the environment condition. The nap environment determination system 1 adjusts the environment condition according to the nap plan. Details will be described below.

### (3-1-5) Update unit 50

The update unit 50 updates the nap plan, based on the intra-nap information while the subject is taking a nap. As described above, the intra-nap information is information acquired while the subject is taking a nap. When the nap plan is updated by the update unit 50, the nap environment determination system 1 adjusts the environment condition of the subject in accordance with the updated nap plan.

### (3-2) Communication unit 220

The communication unit 220 communicates with an external network including the communication network NW1. Through the functioning of the communication unit 220, various data are transmitted and received between the management apparatus 200 and various devices including the pre-nap information acquisition device 60, the intra-nap information acquisition device 70, and the environment condition adjustment device 100.

### (3-3) Storage unit 230

The storage unit 230 includes a medium that records information, such as stored programs and data, so as to be readable by a processor or the like. In the nap environment determination system 1 according to the present embodiment, the CPU or the like constituting the processing unit 210 reads a program stored in the storage unit 230 to realize the functions described above.

The storage unit 230 includes a system memory, such as a random access memory (RAM) or a read only memory (ROM), and an auxiliary storage apparatus. The auxiliary storage apparatus may be, for example, a magnetic recording medium such as a hard disk, an optical recording medium such as a CD or a DVD, or a semiconductor memory such as a flash memory.

### (4) Details of nap plan

Hereinafter, the nap plan according to the present embodiment will be described. The nap plan is a plan related to the environment condition for adjusting the sleep depth of the subject and is a plan created by the creation unit 40.

The creation unit 40 creates the nap plan based on the determination of the determination unit 30. The determination unit 30 determines the environment condition for adjusting the sleep depth of the subject, based on the pre-nap information. Here, it is assumed that, for example, an age, a gender, an average sleep onset time, an average wake-up time, an average sleeping time, drowsiness, a level of fatigue, a sleeping time of the previous day, and a desired nap time, of the subject are acquired as the pre-nap information.

The determination unit 30 determines a nap condition for adjusting the sleep depth of the subject, based on the pre-nap information as described above. For example, the determination unit 30 determines the number of times the environment condition is adjusted. In addition, the determination unit 30 determines, for example, a time at which the environment condition is adjusted. In addition, the determination unit 30 determines how the sleep state transitions, for example. In addition, the determination unit 30 determines how to adjust a control parameter for adjusting the environment condition, for example. Here, the control parameter for adjusting the environment condition includes, for example, at least one of a temperature, a humidity, an airflow, an illuminance, a noise, an odor, a temperature of the bedding, and a gradient of the bedding.

Note that in the present embodiment, the sleep state of the subject includes a REM sleep state, a light sleep state, a deep sleep state, and a wakeful state. Note that the light sleep state here refers to a state in which the subject is in stage 1 or stage 2 of sleep. The deep sleep state refers to a state in which the subject is in stage 3 or stage 4 of sleep. The wakeful state refers to a sleep state that does not correspond to any one of the REM sleep state, the light sleep state, and the deep sleep state.

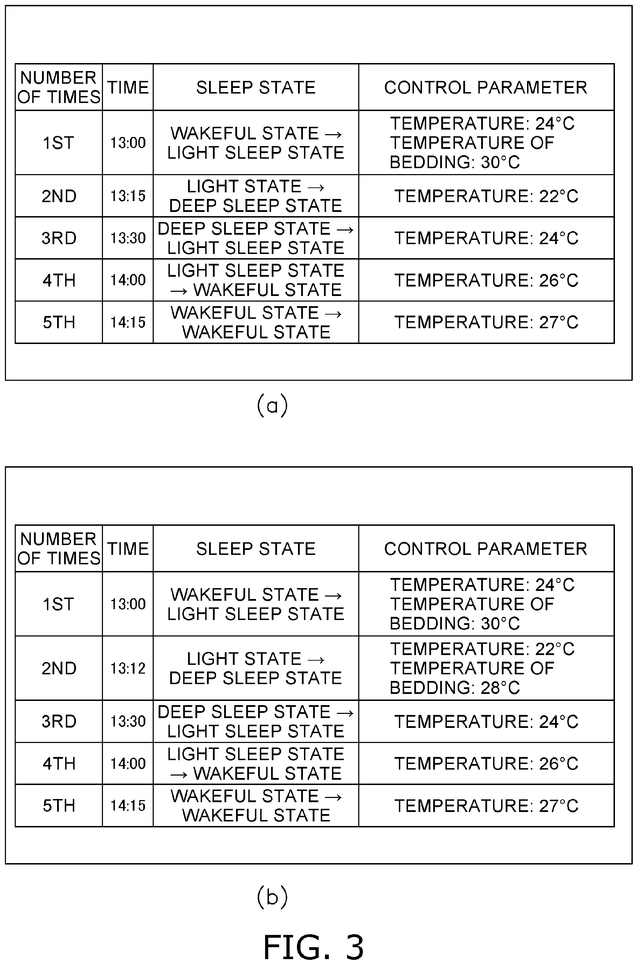

The creation unit 40 creates the nap plan, based on the determination of the determination unit 30 as described above. Figs. 3(a) and 3(b) are examples of the nap plan created by the creation unit 40. Fig. 3(a) illustrates a nap plan immediately after being created by the creation unit 40. Fig. 3(b) illustrates a nap plan updated by the update unit 50. As illustrated in Fig. 3(a), the nap plan immediately after being created by the creation unit 40 includes the number of times the environment condition is adjusted, the times at which the environment condition is adjusted, target transitions of the sleep state switched by adjustment of the environment condition, and control parameters for adjusting the environment condition. The nap environment determination system 1 is scheduled to adjust the sleep depth in accordance with the nap plan created by the creation unit 40.

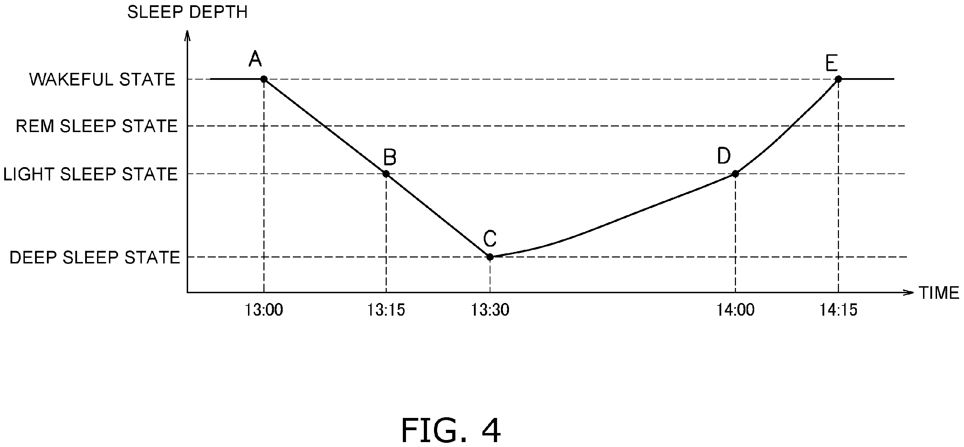

For example, when the nap plan illustrated in Fig. 3(a) is created, the nap environment determination system 1 is scheduled to perform a first adjustment of the environment condition at 13:00 (at the same time as the start of the nap), a second adjustment of the environment condition at 13:15, a third adjustment of the environment condition at 13:30, a fourth adjustment of the environment condition at 14:00, and a fifth adjustment of the environment condition at 14:15. Further, in the first adjustment of the environment condition, the operations of the air-conditioning apparatus 101 and the electric blanket 102a as the environment condition adjustment device 100 are scheduled to be controlled so that the temperature (air temperature in the space RM) becomes 24°C and the temperature of the bedding becomes 30°C. In the second adjustment of the environment condition, the operation of the air-conditioning apparatus 101 is scheduled to be controlled so that the temperature becomes 22°C. In the third adjustment of the environment condition, the operation of the air-conditioning apparatus 101 is scheduled to be controlled so that the temperature becomes 24°C. In the fourth adjustment of the environment condition, the operation of the air-conditioning apparatus 101 is scheduled to be controlled so that the temperature becomes 26°C. In the fifth adjustment of the environment condition, the operation of the air-conditioning apparatus 101 is scheduled to be controlled so that the temperature becomes 27°C. By performing the first adjustment of the environment condition, the sleep state of the subject is expected to switch from the wakeful state to the light sleep state (refer to point A in Fig. 4). By performing the second adjustment of the environment condition, the sleep state of the subject is expected to switch from the light sleep state to the deep sleep state (refer to point B in Fig. 4). By performing the third adjustment of the environment condition, the sleep state of the subject is expected to switch from the deep sleep state to the light sleep state (refer to point C in Fig. 4). By performing the fourth adjustment of the environment condition, the sleep state of the subject is expected to switch from the light state to the wakeful state (refer to point D in Fig. 4). By performing the fifth adjustment of the environment condition, the sleep state of the subject is expected to be maintained in the wakeful state (refer to point E in Fig. 4).

Further, the nap plan according to the present embodiment is updated in real time by the function of the update unit 50 while the subject is taking a nap. As described above, the intra-nap information is acquired while the subject is taking a nap. Here, a case is assumed in which, for example, the heartbeat, breathing, and body temperature of the subject are acquired as the intra-nap information. Note that, here, as the "heartbeat", a heart rate per minute of the subject is acquired. Here, an average number of breaths per minute of the subject is acquired as the "breathing".

By acquiring the intra-nap information described above while the subject is taking a nap, the nap environment determination system 1 can know, for example, that the sleep state of the subject who is taking the nap has switched or that the body temperature of the subject who is taking the nap has increased. The update unit 50 updates the nap plan, based on such intra-nap information. Fig. 3(b) illustrates an example of the updated nap plan. When the nap plan is updated to the contents illustrated in Fig. 3(b), the nap environment determination system 1 changes the schedule so that the second adjustment of the environment condition, which has been scheduled to be performed at 13:15, is performed at 13:12. In addition, in the second adjustment of the environment condition in the updated nap plan, the operation of the electric blanket 102a is controlled so that the temperature of the bedding becomes 28°C. Thus, when the nap plan is updated, the nap environment determination system 1 adjusts the environment condition in accordance with the nap plan updated by the update unit 50.

Note that the nap plan is repeatedly updated based on the intra-nap information acquired in real time. Each time the nap plan is updated, the nap environment determination system 1 adjusts the environment condition in accordance with the updated nap plan.

Note that the contents of the nap plan illustrated in Fig. 3(a) and the contents of the updated nap plan illustrated in Fig. 3(b) are merely examples and can be changed, as appropriate. Therefore, for example, a nap plan may be created in which the lighting device 103, the speaker 104, and the scent dispenser 105 operate in order to adjust the sleep depth.

Thus, in the nap environment determination system 1 according to the present embodiment, the environment condition is adjusted in accordance with the nap plan. More specifically, an environment condition that leads to deep sleep during a nap, an environment condition that leads to light sleep during a nap, and an environment condition that maintains the sleep depth during a nap are determined. Therefore, the nap environment determination system 1 according to the present embodiment can provide an environment that is suitable for the subject during a nap.

In addition, in the nap environment determination system 1 according to the present embodiment, the environment during a nap is controlled in detail in accordance with the nap plan. Therefore, even when the subject takes a short time of sleep, such as a nap, it is possible to provide an environment that is suitable for the subject during a nap.

### (5) Operation of nap environment determination system 1

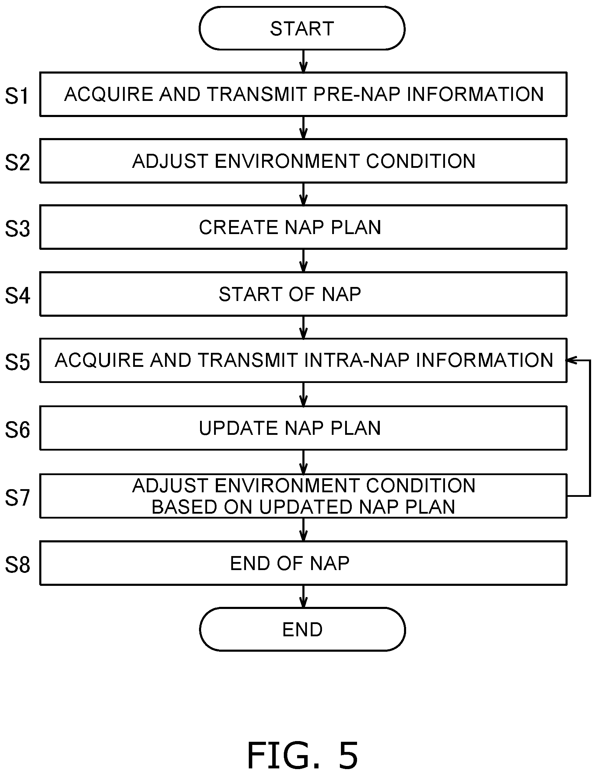

Hereinafter, a flow of the operation of the nap environment determination system 1 according to the present embodiment will be described with reference to Fig. 5. Fig. 5 is a flowchart illustrating an example of the flow of the operation of the nap environment determination system 1. Note that the contents of the steps illustrated in Fig. 5 can be changed, as appropriate. For example, the order of the steps may be changed as long as there is no contradiction; some steps may be executed in parallel with other steps; or other steps may be newly added.

In step S1, the pre-nap information acquisition device 60 of the nap environment determination system 1 acquires pre-nap information and transmits the pre-nap information to the pre-nap information acquisition unit 10.

In step S2, the determination unit 30 of the nap environment determination system 1 determines an environment condition for adjusting the sleep depth of the subject, based on the pre-nap information.

In step S3, the creation unit 40 creates a nap plan, based on the determination of the determination unit 30.

The steps S1 to S3 are performed before a nap of the subject is started.

In step S4, a nap of the subject is started. Here, the nap environment determination system 1 adjusts the environment condition of the subject who takes the nap, based on the nap plan.

In step S5, the intra-nap information acquisition device 70 of the nap environment determination system 1 acquires intra-nap information while the subject is taking the nap and transmits the intra-nap information to the intra-nap information acquisition unit 20.

In step S6, the nap plan is updated by the update unit 50 of the nap environment determination system 1.

In step S7, the nap environment determination system 1 adjusts the environment condition of the subject who is taking the nap, based on the updated nap plan. The nap environment determination system 1 continues the processing from step S5 to step S7 until the nap of the subject ends.

In step S8, the nap of the subject ends.

In this way, the nap environment determination system 1 determines the environment condition during a nap.

### (6) Features

PTL 1 noted above discloses a sleep state improvement system and a sleep state improvement method capable of improving the sleep state of a user (subject) also while the user is away from home. However, in PTL 1 noted above, a case in which the user takes a nap is not considered in detail. Therefore, the sleep environment provided by the system and method of PTL 1 noted above is not necessarily an environment suitable for the user who takes a nap.

### (6-1)

The nap environment determination system 1 according to the present embodiment is a system for determining an environment during a nap and includes the pre-nap information acquisition unit 10 and the determination unit 30. The pre-nap information acquisition unit 10 acquires pre-nap information regarding a subject who takes a nap before the subject takes the nap. The determination unit 30 determines an environment condition for adjusting the sleep depth, based on the pre-nap information.

In the nap environment determination system 1, which is a system for determining an environment condition during a nap, the determination unit 30 determines an environment condition for adjusting the sleep depth, based on the pre-nap information. The pre-nap information is information regarding the subject who takes a nap and is information acquired before the subject takes the nap. According to this configuration, the nap environment determination system 1 can determine the environment condition during a nap, based on the environment condition for adjusting the sleep depth which is determined by the determination unit 30. More specifically, it is possible to determine an environment condition that increases the sleep depth of the subject during a nap, an environment condition that decreases the sleep depth of the subject during a nap, or an environment condition that maintains the sleep depth of the subject during a nap. Therefore, the nap environment determination system 1 according to the present embodiment can provide an environment that is suitable for the subject during a nap.

### (6-2)

The nap environment determination system 1 according to the present embodiment further includes the creation unit 40. The creation unit 40 creates a nap plan related to the environment condition for adjusting the sleep depth, based on the determination of the determination unit 30. The nap plan includes the number of times the environment condition is adjusted, a time at which the environment condition is adjusted, a target transition of a sleep state switched by adjustment of the environment condition, and a control parameter for adjusting the environment condition.

In the nap environment determination system 1, the creation unit 40 creates a nap plan related to the environment condition for adjusting the sleep depth, based on the determination of the determination unit 30. According to this configuration, the nap environment determination system 1 can determine the environment condition during a nap, based on the nap plan created by the creation unit 40. Therefore, the nap environment determination system 1 according to the present embodiment can provide an environment that is suitable for the subject during a nap.

### (6-3)

The nap environment determination system 1 according to the present embodiment further includes the intra-nap information acquisition unit 20 and the update unit 50. The intra-nap information acquisition unit 20 acquires intra-nap information regarding the subject who is taking a nap while the subject is taking the nap. The update unit 50 updates the nap plan, based on the intra-nap information while the subject is taking the nap.

In the nap environment determination system 1, the update unit 50 updates the nap plan, based on the intra-nap information while the subject is taking a nap. The intra-nap information is information regarding the subject who is taking a nap and is information acquired while the subject is taking the nap. According to this configuration, the nap environment determination system 1 can determine the environment condition during a nap, based on the nap plan that has been updated while the subject is taking the nap. Therefore, the nap environment determination system 1 according to the present embodiment can provide an environment that is more suitable for the subject during a nap.

### (6-4)

In the nap environment determination system 1 according to the present embodiment, the pre-nap information includes at least one of a sleep onset time of a previous day, an average sleep onset time, a wake-up time of the previous day, an average wake-up time, and drowsiness, of the subject. In addition, the pre-nap information includes at least one of a gender, an age, and a desired nap time, of the subject.

In the nap environment determination system 1, information specific to the subject is acquired as the pre-nap information. According to this configuration, the nap environment determination system 1 can determine the environment condition during a nap in accordance with the characteristics of the subject. Therefore, the nap environment determination system 1 according to the present embodiment can provide an environment that is more suitable for the subject during a nap.

### (6-5)

In the nap environment determination system 1 according to the present embodiment, the pre-nap information further includes at least one of a level of fatigue, a sleeping time of a previous day, and an average sleeping time, of the subject.

In the nap environment determination system 1, information specific to the subject is acquired as the pre-nap information. According to this configuration, the nap environment determination system 1 can determine the environment condition during a nap in accordance with the characteristics of the subject. Therefore, the nap environment determination system 1 according to the present embodiment can provide an environment that is more suitable for the subject during a nap.

### (6-6)

In the nap environment determination system 1 according to the present embodiment, the pre-nap information is acquired from at least one of a manual input by the subject and the pre-nap information acquisition device 60 that is a device for acquiring information about the subject.

### (6-7)

In the nap environment determination system 1 according to the present embodiment, the intra-nap information includes at least one of a heartbeat, an electrocardiogram, a pulse wave, a brain wave, breathing, a blood pressure, a body temperature, and a body motion. In addition, the intra-nap information includes at least one of a temperature, a humidity, an airflow, an illuminance, a noise, an odor, a temperature of the bedding, and a gradient of the bedding, in a space RM in which the subject who is taking a nap is present. The intra-nap information is acquired by the intra-nap information acquisition device 70 installed in the space in which the subject who is taking the nap is present.

In the nap environment determination system 1, biological information specific to the subject and information specific to the space RM in which the subject is present are acquired as the intra-nap information. In addition, the intra-nap information is information acquired while the subject is taking a nap. According to this configuration, the nap environment determination system 1 can determine the environment condition during a nap, based on the current characteristics of the subject and the current characteristics of the space RM in which the subject is present. Therefore, the nap environment determination system 1 according to the present embodiment can provide an environment that is more suitable for the subject during a nap.

### (6-8)

In the nap environment determination system 1 according to the present embodiment, the sleep state includes a wakeful state, a REM sleep state, a light sleep state, and a deep sleep state.

### (6-9)

In the nap environment determination system 1 according to the present embodiment, the environment condition includes at least one of a thermal environment, a lighting environment, an intra-bedding thermal environment, a bedding position, and a sound environment, in the space in which the subject who is taking a nap is present.

### (6-10)

The nap environment determination system 1 according to the present embodiment further includes the environment condition adjustment device 100 and the actuator 110. The environment condition adjustment device 100 is a device for adjusting the environment condition, based on the nap plan. The actuator 110 controls the environment condition adjustment device 100. The environment condition adjustment device 100 includes the air-conditioning apparatus 101 and the bedding 102 having a temperature adjustment unit.

The nap environment determination system 1 further includes the environment condition adjustment device 100 that is a device for adjusting the environment condition, based on the nap plan, and the actuator 110 that controls the environment condition adjustment device 100. According to this configuration, the environment condition can be adjusted by the environment condition adjustment device 100.

### (7) Modifications

The embodiment described above can be appropriately modified as illustrated in the following modifications. Each modification may be applied in combination with another modification within a range such that no contradiction occurs. Note that the same configurations as those in the first embodiment are denoted by the same reference numerals, and detailed description thereof will be omitted.

### (7-1) Modification 1A

The nap environment determination system 1 according to the present disclosure may further include an output unit that outputs the nap plan. Alternatively, for example, the pre-nap information acquisition device 60 or the management apparatus 200 may have a function as the output unit.

In the nap environment determination system 1 according to the present modification, for example, the subject can check the nap plan immediately after being created by the creation unit 40, and a history of updates to the nap plan. Therefore, it is possible to easily confirm how the user's nap environment is determined.

### (7-2) Modification 1B

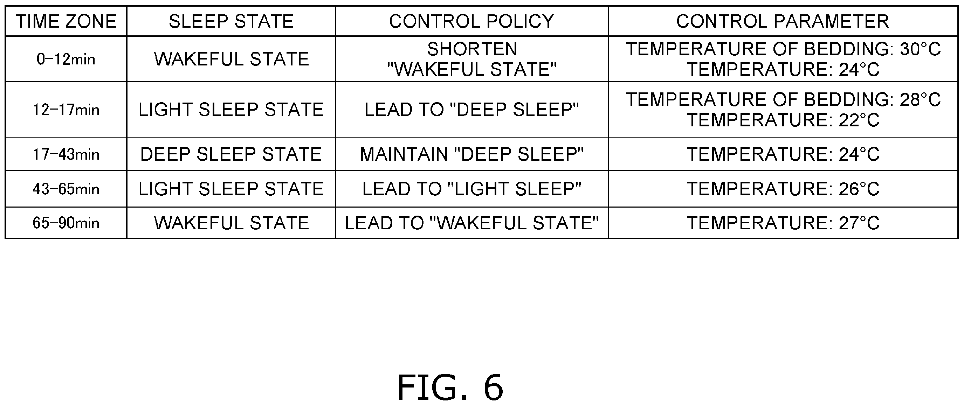

The nap environment determination system 1 according to the present disclosure may create a report in which items related to the nap of the subject are described in detail. Hereinafter, such a report is referred to as a nap report. In addition, when the nap environment determination system 1 includes the output unit described in the modification 1A, the nap report may be outputted by the output unit. For example, the nap environment determination system 1 may create and output a nap report as illustrated in Fig. 6.

As illustrated in Fig. 6, the nap report may include a time zone in which a nap is taken, a sleep state in each time zone, a control policy in each time zone, control parameters in each time zone, and the like. In addition, the nap report may include various kinds of information related to a nap of the subject, such as the pre-nap information acquired before a nap and the intra-nap information acquired during a nap.

In the nap environment determination system 1 according to the present modification, for example, the subject can easily check his/her own biological information during a nap, a transition of his/her own sleep state, and the like.

### (7-3) Modification 1C

While in the embodiment described above, the management apparatus 200 is described as being installed in a space different from the space RM, this is not a limitation. For example, the management apparatus 200 may be installed inside the space RM. In this case, the management apparatus 200 may perform, for example, short-range wireless communication with the pre-nap information acquisition device 60, the intra-nap information acquisition device 70, and the environment condition adjustment device 100 to receive and transmit various data.

### <Other Embodiments>

While the embodiments of the present disclosure have been described, it will be understood that various changes in form or details may be made therein without departing from the spirit and scope of the claims.

The present disclosure is not limited to the embodiments described above as they are. The present disclosure can be embodied by modifying its constituent elements without departing from the gist of the present disclosure in the implementation stage. In addition, the present disclosure can form various disclosures by appropriately combining a plurality of constituent elements disclosed in each of the embodiments described above. For example, some constituent elements may be deleted from all the constituent elements illustrated in the embodiments. Further, constituent elements may be appropriately combined in different embodiments. The present embodiments are, therefore, to be considered in all respects as illustrative and not restrictive, and all modifications apparent to those skilled in the art are intended to be included therein.

### Reference Signs List

- 1: Nap environment determination system
- 10: Pre-nap information acquisition unit
- 20: Intra-nap information acquisition unit
- 30: Determination unit
- 40: Creation unit
- 50: Update unit
- 60: Pre-nap information acquisition device
- 70: Intra-nap information acquisition device
- 100: Environment condition adjustment device
- 101: Air-conditioning apparatus
- 102: Bedding
- 110: Actuator

### Citation List

### Patent Literature

PTL 1: Japanese Patent No. 4466654

## Claims

1. A nap environment determination system (1) that is a system for determining an environment condition during a nap, the system comprising:
a pre-nap information acquisition unit (10) that acquires pre-nap information regarding a subject who takes a nap before the subject takes the nap; and
a determination unit (30) that determines an environment condition for adjusting a sleep depth, based on the pre-nap information.

2. The nap environment determination system according to claim 1, further comprising a creation unit (40) that creates a nap plan related to the environment condition for adjusting the sleep depth, based on a determination of the determination unit, wherein
the nap plan includes a number of times the environment condition is adjusted, a time at which the environment condition is adjusted, a target transition of a sleep state switched by adjustment of the environment condition, and a control parameter for adjusting the environment condition.

3. The nap environment determination system according to claim 1 or 2, further comprising:
an intra-nap information acquisition unit (20) that acquires intra-nap information regarding the subject who is taking a nap while the subject is taking the nap; and
an update unit (50) that updates the nap plan, based on the intra-nap information while the subject is taking the nap.

4. The nap environment determination system according to any one of claims 1 to 3, wherein
the pre-nap information includes
at least one of a sleep onset time of a previous day, an average sleep onset time, a wake-up time of the previous day, an average wake-up time, and drowsiness, of the subject, and
at least one of a gender, an age, and a desired nap time, of the subject.

5. The nap environment determination system according to any one of claims 1 to 4, wherein the pre-nap information further includes at least one of a level of fatigue, a sleeping time of a previous day, and an average sleeping time, of the subject.

6. The nap environment determination system according to any one of claims 1 to 5, wherein
the pre-nap information is acquired from at least one of a manual input by the subject and a pre-nap information acquisition device (60) that is a device for acquiring information about the subject.

7. The nap environment determination system according to any one of claims 3 to 6, wherein
the intra-nap information includes
at least one of a heartbeat, an electrocardiogram, a pulse wave, a brain wave, breathing, a blood pressure, a body temperature, and a body motion, and
at least one of a temperature, a humidity, an airflow, an illuminance, a noise, an odor, a temperature of bedding, and a gradient of the bedding, in a space in which the subject who is taking a nap is present, and
the intra-nap information is acquired by an intra-nap information acquisition device (70) installed in the space in which the subject who is taking the nap is present.

8. The nap environment determination system according to any one of claims 2 to 7, wherein
the sleep state includes a wakeful state, a REM sleep state, a light sleep state, and a deep sleep state.

9. The nap environment determination system according to any one of claims 2 to 8, wherein
the environment condition includes at least one of a thermal environment, a lighting environment, an intra-bedding thermal environment, a bedding position, and a sound environment, in a space in which the subject who is taking a nap is present.

10. The nap environment determination system according to any one of claims 2 to 9, further comprising:
an environment condition adjustment device (100) that is a device for adjusting the environment condition, based on the nap plan; and
an actuator (110) that controls the environment condition adjustment device, wherein
the environment condition adjustment device includes an air-conditioning apparatus (101) and bedding (102) having a temperature adjustment unit.
